# EUROPEAN PATENT APPLICATION

(11) **EP 4 793 345 A1**
(43) Date of publication of application: **19.08.2026**
(21) Application number: 24902902.6
(22) Date of filing: 12.12.2024
(51) Int. Cl.: C12N 1/14, C12N 15/01, C12Q 1/04, C12R 1/645

(54) **SCHIZOCHYTRIUM ALGAE STRAIN RICH IN N-3 FATTY ACID AND SCREENING AND CULTURE METHOD THEREFOR**

(30) Priority: 14.12.2023 CN 202311728644
(71) Applicant: Xiamen Huison Biotech Co., Ltd., Xiamen, Fujian 361000 (CN)
(72) Inventor: CHEN, Liyi, Xiamen, Fujian 361000 (CN); ZHONG, Huichang, Xiamen, Fujian 361000 (CN); CHEN, Shuirong, Xiamen, Fujian 361000 (CN); ZENG, Shengli, Xiamen, Fujian 361000 (CN)
(74) Representative: Metida
(86) International application number: PCT/CN2024/138836
(87) International publication number: WO 2025/124485

(57) **Abstract**

The present invention belongs to the field of biotechnology. The present invention provides a Schizochytrium strain rich in n-3 fatty acids and screening and cultivation methods therefor. Compared with the original strains, the strain provided in the present invention has significantly increased yields of EPA and n-3 DPA, and the strain features good genetic stability, a fast growth rate and promising prospects for industrial application. The present invention also provides a medium for mutagenizing a Schizochytrium sp. and a medium for screening a Schizochytrium sp., as well as a suitable mutagenesis method and screening method.

## Description

### TECHNICAL FIELD

The present invention relates to the field of biotechnology, and specifically, to a Schizochytrium strain rich in n-3 fatty acids and screening and cultivation methods therefor.

### TECHNICAL BACKGROUND

n-3 fatty acids are a class of polyunsaturated fatty acids, mainly including eicosapentaenoic acid (C20:5, cis-5,8,11,14,17, EPA), docosapentaenoic acid (C22:5, cis-7,10,13,16,19, n-3 DPA), docosahexaenoic acid (C22:6, cis-4,7,10,13,16,19, DHA), and the like, which exist in deep-sea marine animals and marine microorganisms. As essential nutritional active substances for humans, n-3 fatty acids are crucial in human neural development and prevention of chronic diseases. Conventional sources of n-3 fatty acids are fish oil. However, due to various factors such as the increasing depletion of marine resources and frequent marine pollution, the supply of high-quality fish oil has decreased significantly. As the primary producers of n-3 fatty acids, marine microalgae feature safe and controllable fermentation processes without occupying land resources, which makes marine microalgae a green and sustainable alternative to fish oil.

Schizochytrium sp. is one of the most important oleaginous microalgae in current commercial production, mainly used for DHA production with a yield of up to 40 g/L. In addition, Schizochytrium can also produce unsaturated fatty acids such as EPA and n-3 DPA simultaneously. However, due to their limited ability to synthesize EPA and n-3 DPA, wild-type strains cannot meet requirements for industrial application. Therefore, exploring Schizochytrium having also a high yield of n-3 fatty acids has long been a study hotspot for relevant researchers. Numerous studies have shown that the fatty acid biosynthesis pathways in Schizochytrium mainly include a fatty acid synthase pathway and a polyketide synthase pathway. The fatty acid synthase pathway is found in most microorganisms, mainly involving a series of steps such as dehydration, condensation and elongation of fatty acid precursors. In fatty acid synthesis, acetyl-CoA and malonyl-CoA first react to produce palmitic acid (C16:00), palmitic acid is then converted into linoleic acid (C18:2n-6) under the catalysis of elongases and desaturases, and linoleic acid further enters an n-6 pathway and n-3 pathway under the catalysis of Δ15 and Δ6 desaturases, respectively. Unsaturated fatty acids such as EPA, n-3 DPA, and DHA can be ultimately synthesized through the n-3 pathway. The polyketide synthase pathway mainly involves dehydration and isomerization reactions of fatty acid acyl intermediates. Studies have shown that the polyketide synthase pathway can be directly used for synthesis of not only DHA, but also DPA and EPA, although the specific synthetic pathways and mechanisms remain controversial.

Therefore, there is a strong demand for exploring ideal approaches such as strain mutagenesis, strain domestication and genetic engineering to enhance the n-3 pathway of fatty acid synthases in Schizochytrium, thereby increasing the yields of EPA and n-3 DPA.

### Summary of Invention

To resolve the above technical problems, the present invention provides a medium for mutagenizing a Schizochytrium sp. and a medium for screening a Schizochytrium sp. A strain of Schizochytrium sp. is obtained by a suitable mutagenesis method and screening method. Compared with original strains, the strain has significantly increased yields of EPA and DPA, and the strain features good genetic stability, a fast growth rate and promising prospects for industrial application.

According to a first aspect, the present invention provides a strain of Schizochytrium sp., designated as Schizochytrium sp. HS08, with deposit information: deposited at the China General Microbiological Culture Collection Center (CGMCC) under the accession number CGMCC No. 40902. Proportions of EPA and n-3 DPA in the total fatty acids of the fermentation broth produced by the Schizochytrium sp. HS08 are significantly higher, exhibiting unexpected technical effects. In addition, after multiple subcultures of the strain provided in the present invention, the obtained strains can stably produce various n-3 fatty acids, and have advantages of good genetic stability, a fast growth rate and promising prospects for industrial application.

According to a second aspect, the present invention provides a fermentation broth.

According to a third aspect, the present invention provides a Schizochytrium suspension.

According to a fourth aspect, the present invention provides a microbial agent.

According to a fifth aspect, the present invention provides a method for preparing the fermentation broth according to the second aspect.

According to a sixth aspect, the present invention provides a method for preparing an n-3 fatty acid.

According to a seventh aspect, the present invention provides an n-3 fatty acid.

According to an eighth aspect, the present invention provides a method for preparing a microbial oil containing an n-3 fatty acid.

According to a ninth aspect, the present invention provides a microbial oil containing an n-3 fatty acid.

According to a tenth aspect, the present invention provides use of the foregoing Schizochytrium sp., fermentation broth, Schizochytrium suspension, microbial agent, the fermentation broth prepared in the foregoing preparation method, the n-3 fatty acid prepared in the foregoing preparation method, the n-3 fatty acid, the microbial oil containing an n-3 fatty acid prepared in the foregoing preparation method, and the microbial oil containing an n-3 fatty acid.

According to an eleventh aspect, the present invention provides a mutagenic medium for mutagenizing a Schizochytrium sp.

According to a twelfth aspect, the present invention provides a screening medium for screening a Schizochytrium sp.

According to a thirteenth aspect, the present invention provides a method for screening a Schizochytrium sp.

### Detailed Description of Invention

To resolve the above technical problems, the present invention provides a medium for mutagenizing a Schizochytrium sp. and a medium for screening a Schizochytrium sp. A strain of Schizochytrium sp. is obtained by a suitable mutagenesis method and screening method. Compared with original strains, the strain has significantly increased yields of EPA and DPA, and the strain features good genetic stability, a fast growth rate and promising prospects for industrial application.

Specifically, according to a first aspect, the present invention provides a strain of Schizochytrium sp., designated as Schizochytrium sp. HS08, with deposit information: deposited at the China General Microbiological Culture Collection Center (CGMCC) under the accession number CGMCC No. 40902.

According to a second aspect, the present invention provides a fermentation broth.

The fermentation broth is prepared by fermenting the Schizochytrium sp. according to the first aspect.

In some embodiments, the proportion of EPA in the total fatty acids in the fermentation broth is at least 9%. In some embodiments, the proportion of EPA in the total fatty acids in the fermentation broth is 9% to 10%. In some embodiments, the proportion of EPA in the total fatty acids in the fermentation broth is 9%, 9.1%, 9.2%, 9.3%, 9.4%, 9.5%, 9.6%, 9.7%, 9.8%, 9.9%, or 10%.

In some embodiments, the proportion of n-3DPA in the total fatty acids in the fermentation broth is at least 5%. In some embodiments, the proportion of n-3 DPA in the total fatty acids in the fermentation broth is 5%, 5.5%, 6%, 6.5%, or 7%.

In some embodiments, the proportion of DHA in the total fatty acids in the fermentation broth is at least 35%. In some embodiments, the proportion of DHA in the total fatty acids in the fermentation broth is 35%, 36%, 37%, 38%, 39%, 40%, 41%, 42%, 43%, 44%, or 45%.

In some embodiments, the fermentation broth is a mixture of one or more of the Schizochytrium sp., sugar, a protein, an amino acid, and an inorganic salt; and a dry weight proportion of biomass cells of the Schizochytrium sp. in the fermentation broth is at least 5%.

In some embodiments, oil in the Schizochytrium sp. in the fermentation broth accounts for at least 1%, 2%, 3%, 4%, 5%, or 6% of the mixture.

In some embodiments, the oil includes one or more of total fatty acids, phospholipids, liposoluble substances, and sterols; the total fatty acids include at least one of saturated fatty acids, unsaturated fatty acids, and n-3 fatty acids. In some embodiments, a mass ratio of total fatty acids in the oil may be 85%, 86%, 87%, 88%, 89%, 90%, 91%, 92%, 93%, 94%, 95%, or 96%.

In some embodiments, the proportion of oil in the dry weight of biomass cells is at least 50%.

In some embodiments, the proportion of n-3 fatty acid contained in the oil in the dry cell mass is at least 42%.

In some embodiments, the proportion of n-3 fatty acid contained in the oil relative to the total fatty acids is at least 50%. In some embodiments, the proportion of n-3 fatty acid contained in the oil relative to the total fatty acids is at least 55%.

In some embodiments, the oil in the Schizochytrium sp. accounts for at least 1% to 10% of the mixture, the proportion of the oil in the dry weight of biomass cells is at least 50%, and the proportion of n-3 fatty acid contained in the oil relative to the dry cell mass is at least 45%.

In some embodiments, EPA accounts for at most 9% of the oil.

In some embodiments, EPA accounts for at most 10% of the oil; and DHA accounts for at least 35%.

In some embodiments, EPA accounts for at most 10% of the oil; and n-3DPA accounts for at least 5%.

In some embodiments, EPA accounts for at most 10% of the oil; DHA accounts for at least 40%; and n-3DPA accounts for at least 5%.

In some embodiments, a ratio of DHA to EPA in the fatty acids is not less than 4:1.

In some embodiments, a ratio of DHA to n-3 DPA in the fatty acids is not less than 8:1.

In some embodiments, the ratio of DHA to EPA in the fatty acids is not less than 4:1; and the ratio of DHA to n-3 DPA in the fatty acids is not less than 6:1.

According to a third aspect, the present invention provides a Schizochytrium suspension.

The Schizochytrium suspension includes the Schizochytrium sp. according to the first aspect.

According to a fourth aspect, the present invention provides a microbial agent.

The microbial agent includes at least one of the Schizochytrium sp. according to the first aspect, the fermentation broth according to the second aspect, and the Schizochytrium suspension according to the third aspect.

According to a fifth aspect, the present invention provides a method for preparing the fermentation broth according to the second aspect.

The method for preparing the fermentation broth according to the second aspect includes: subjecting, to seed culture and fermentation culture, the Schizochytrium sp. according to the first aspect.

In some embodiments, the seed culture includes: culturing the Schizochytrium sp. HS08 in a seed medium to obtain an inoculum.

In some embodiments, the fermentation culture includes: adding the inoculum obtained after the seed culture of the Schizochytrium sp. HS08 into a fermentation medium for fermentation culture.

In some embodiments, the seed medium includes the following components: 50 g/L glucose, 10 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate and 0.17 g/L anhydrous calcium chloride.

In some embodiments, the fermentation medium includes the following components: 50 g/L glucose, 12 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate, 0.17 g/L anhydrous calcium chloride and 10 g/L agar powder.

In some embodiments, water is used as a solvent in the seed culture medium.

In some embodiments, water is used as a solvent in the fermentation medium.

In some embodiments, pH of the seed culture medium is not additionally adjusted (that is, the pH is natural).

In some embodiments, pH of the fermentation medium is not additionally adjusted (that is, the pH is natural).

In some embodiments, culture time for the seed culture is 12 hours to 60 hours. In some embodiments, culture time for the seed culture is 12, 15, 20, 24, 25, 30, 35, 40, 45, 46, 47, 48, 49, 50, 55, or 60 hours. In some embodiments, the culture time for the seed culture is 48 hours.

In some embodiments, culture temperature for the seed culture is 20°C to 30°C. In some embodiments, the culture temperature for the seed culture is 20°C, 21°C, 22°C, 23°C, 24°C, 25°C, 26°C, 27°C, 28°C, 29°C, or 30°C.

In some embodiments, a rotation speed for the seed culture is 100 rpm to 250 rpm. In some embodiments, the rotation speed for the seed culture is 100 rpm, 110 rpm, 120 rpm, 130 rpm, 140 rpm, 150 rpm, 160 rpm, 170 rpm, 180 rpm, 190 rpm, 200 rpm, 210 rpm, 220 rpm, 230 rpm, 240 rpm, or 250 rpm.

In some embodiments, culture time for the fermentation culture is 100 hours to 300 hours. In some embodiments, the culture time for the fermentation culture is 100, 110, 120, 130, 140, 150, 200, 250, or 300 hours.

In some embodiments, culture temperature for the fermentation culture is 25°C to 30°C. In some embodiments, the culture temperature for the seed culture is 25°C, 25.5°C, 26°C, 26.5°C, 27°C, 27.5°C, 28°C, 28.5°C, 29°C, 29.5°C, or 30°C.

In some embodiments, the rotation speed for the fermentation culture is 150 rpm to 200 rpm. In some embodiments, the rotation speed for the seed culture is 150 rpm, 160 rpm, 170 rpm, 180 rpm, 190 rpm, or 200 rpm.

According to a sixth aspect, the present invention provides a method for preparing an n-3 fatty acid.

The method for preparing an n-3 fatty acid includes: extracting the n-3 fatty acid from the fermentation broth according to the second aspect or the fermentation broth prepared in the preparation method according to the fifth aspect.

In some embodiments, the n-3 fatty acid includes at least one of EPA, n-3 DPA and DHA.

According to a seventh aspect, the present invention provides an n-3 fatty acid.

The n-3 fatty acid is prepared in the preparation method according to the sixth aspect.

According to an eighth aspect, the present invention provides a method for preparing a microbial oil containing an n-3 fatty acid.

The method for preparing a microbial oil containing an n-3 fatty acid includes: extracting the microbial oil containing an n-3 fatty acid from the fermentation broth according to the second aspect or the fermentation broth prepared in the preparation method according to the fifth aspect.

In some embodiments, the microbial oil containing an n-3 fatty acid includes at least one of EPA, n-3 DPA and DHA.

According to a ninth aspect, the present invention provides a microbial oil containing an n-3 fatty acid.

The microbial oil containing an n-3 fatty acid is prepared in the preparation method according to the eight aspect.

According to a tenth aspect, the present invention provides use of the foregoing Schizochytrium sp., fermentation broth, Schizochytrium suspension, microbial agent, the fermentation broth prepared in the foregoing preparation method, the n-3 fatty acid prepared in the foregoing preparation method, the n-3 fatty acid, the microbial oil containing an n-3 fatty acid prepared in the foregoing preparation method, and the microbial oil containing an n-3 fatty acid.

The Schizochytrium sp. according to the first aspect, the fermentation broth according to the second aspect, the Schizochytrium suspension according to the third aspect, the microbial agent according to the fourth aspect, the fermentation broth prepared in the preparation method according to the fifth aspect, the n-3 fatty acid prepared in the preparation method according to the sixth aspect, the n-3 fatty acid according to the seventh aspect, the microbial oil containing an n-3 fatty acid prepared in the preparation method according to the eighth aspect, or the microbial oil containing an n-3 fatty acid according to the ninth aspect is used in preparation of food, a cosmetic, a health product, a feed, and/or a medicament.

According to an eleventh aspect, the present invention provides a mutagenic medium for mutagenizing a Schizochytrium sp., where the mutagenic medium contains a mutagen selected from one or more of nitrosoguanidine, ethyl methanesulfonate and diethyl sulfate; and a concentration of the mutagen is 0.1% to 5.0%.

In some embodiments, the mutagen is 0.5%~2% ethyl methanesulfonate or 0.5%~2% diethyl sulfate; or preferably, the mutagen is 1% ethyl methanesulfonate or 1% diethyl sulfate.

In some embodiments, the mutagenic medium further contains 40-60 g/L glucose, 9-15 g/L yeast extract powder, 15-20 g/L sodium sulfate anhydrous, 0.5-1 g/L potassium chloride, 3-5 g/L magnesium sulfate heptahydrate, 0.25-0.9 g/L potassium sulfate, 0.5-3.5 g/L potassium dihydrogen phosphate, 0.5-3.5 g/L ammonium sulfate and 0.1-2 g/L anhydrous calcium chloride, with natural pH.

In some embodiments, the mutagenic medium includes: 50 g/L glucose, 10 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate and 0.17 g/L anhydrous calcium chloride.

According to a twelfth aspect, the present invention provides a screening medium for screening a Schizochytrium sp., where the screening medium contains a screening agent selected from one or more of quizalofop-p-ethyl, cerulenin, VB12 and diphenylamine; and a concentration of cerulenin is 0.001-1 mM, a concentration of quizalofop-p-ethyl is 0.001-1 mM, a concentration of diphenylamine is 0.001-1 mM, and a concentration of VB12 is 1-100 µg/L.

In some embodiments, the concentration of quizalofop-p-ethyl is 0.01-0.08 mM; the concentration of cerulenin is 0.01-0.02 mM; and the concentration of VB12 is 0.01 µg/L.

In some embodiments, the screening medium further contains 40-60 g/L glucose, 9-15 g/L yeast extract powder, 15-20 g/L sodium sulfate anhydrous, 0.5-1 g/L potassium chloride, 3-5 g/L magnesium sulfate heptahydrate, 0.25-0.9 g/L potassium sulfate, 0.5-3.5 g/L potassium dihydrogen phosphate, 0.5-3.5 g/L ammonium sulfate, 0.1-2 g/L anhydrous calcium chloride, and 5-20 g/L agar powder, with natural pH.

In some embodiments, the screening medium contains 50 g/L glucose, 12 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate, 0.17 g/L anhydrous calcium chloride, and 10 g/L agar powder.

According to a thirteenth aspect, the present invention provides a method for screening a Schizochytrium sp., including steps of:
(1) inoculating the Schizochytrium sp. into a seed medium for cultivation for 24 hours to obtain a Schizochytrium inoculum;
(2) pipetting and centrifuging the seed solution and discarding a supernatant; washing and resuspending a residue with a phosphate buffer at pH 8.0 to obtain a seed suspension; and inoculating the seed suspension into the mutagenic medium in the present invention to obtain a mutated Schizochytrium solution;
(3) pipetting the mutated Schizochytrium solution, and diluting the mutated Schizochytrium solution to a certain gradient with a phosphate buffer at pH 8.0, and then pipetting the diluted mutated Schizochytrium solution to spread on the screening medium in the present invention for cultivation;
(4) selecting single colonies with fast growth rates and regular edges, and streaking the single colonies onto the screening medium in the present invention for cultivation; and
(5) selecting colonies with fast growth rates and moderate size, inoculating the colonies into a seed medium, culturing for 24 hours and then preserving, to obtain the candidate purified high-yield Schizochytrium sp.

In step (2), washing and resuspending with the phosphate buffer at pH 8.0 means washing with the phosphate buffer at pH 8.0 and resuspending with the phosphate buffer at pH 8.0.

In some embodiments, the Schizochytrium sp. is Schizochytrium sp. HS01, deposited at the China General Microbiological Culture Collection Center (CGMCC) under the accession number CGMCC No. 13746.

In some embodiments, the seed medium contains 40-60 g/L glucose, 9-15 g/L yeast extract powder, 15-20 g/L sodium sulfate anhydrous, 0.5-1 g/L potassium chloride, 3-5 g/L magnesium sulfate heptahydrate, 0.25-0.9 g/L potassium sulfate, 0.5-3.5 g/L potassium dihydrogen phosphate, 0.5-3.5 g/L ammonium sulfate and 0.1-2 g/L anhydrous calcium chloride, with natural pH.

In some embodiments, the seed medium contains 50 g/L glucose, 12 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate and 0.17 g/L anhydrous calcium chloride.

In some embodiments, in step (2), the colonies are cultured at 28°C and 180 r/min for 1-6 hours.

In some embodiments, in step (3), the colonies are cultured upside down in an incubator at 37°C for 48 hours.

In some embodiments, in step (4), the colonies are cultured upside down in an incubator at 37°C for 48 hours.

In some embodiments, in step (5), the colonies are cultured on a shaker at 28°C and 180 r/min for 24 hours.

### Beneficial effects

Compared with the prior art, the strain provided in the present invention has the following beneficial technical effects:
(1) Proportions of EPA and n-3 DPA in total fatty acids of the fermentation broth obtained by fermentation of the strain provided in the present invention are significantly higher, exhibiting an unexpected technical effect.
(2) After multiple subcultures of the strain provided in the present invention, the obtained strains can stably produce various n-3 fatty acids, and have advantages of good genetic stability, a fast growth rate and promising prospects for industrial application.

### Terminology Explanation

In the description of this specification, descriptions made with reference to the terms "an embodiment", "some embodiments", "example", "specific example", or "some examples" mean that the specific features, structures, materials or characteristics described with reference to the embodiment or example are included in at least one embodiment or example of the present invention. In this specification, the schematic descriptions of the above terms do not necessarily refer to the same embodiment or example. In addition, the described specific features, structures, materials or characteristics may be suitably combined in any one or more embodiments or examples. In addition, a person skilled in the art can combine and integrate the different embodiments or examples described in this specification, as well as the features of the different embodiments or examples, provided that they do not contradict each other.

In the following descriptions, all numerical values disclosed herein are approximate values, irrespective of whether terms such as "approximately" or "about" are used. A value of each numerical figure may deviate by 1%, 2%, 5%, 7%, 8%, 10%, 15%, or 20%. Whenever a number with a value of N is disclosed, any number with a value of N ±1%, N ±2%, N ±3%, N ±5%, N ±7%, N ±8%, N ±10%, N ±15% or N ±20% is explicitly disclosed, where "+/-" means plus or minus.

### DETAILED DESCRIPTION OF EMBODIMENTS

For a clearer understanding of the objectives, technical solutions and advantages of the present invention, the present invention is further described in detail below with reference to the embodiments. The specific embodiments described herein are only intended to illustrate the present invention rather than constitute any limitation to the present invention. In addition, in the following description, descriptions of well-known structures and technologies are omitted to avoid unnecessarily obscuring concepts of this disclosure. Such structures and techniques have also been described in numerous publications.

All reagents used in the present invention are either commercially available or can be prepared in the methods described herein.

In the present invention, the original strain Schizochytrium sp. HS01 (deposited at the China General Microbiological Culture Collection Center (CGMCC) under the accession number CGMCC No. 13746) producing polyunsaturated fatty acids is used as a starting strain. The proportion of DHA in the total fatty acids of the strain can reach at least 40%, but proportions of EPA and n-3 DPA are only approximately 0.5%. It is intended to obtain new strains rich in EPA, n-3 DPA and DHA through mutagenesis and screening methods.

### Media:

Unless otherwise specified, formulations of the media used in the examples or comparative examples of the present invention are as follows:
Formulation of a seed medium: 50 g/L glucose, 10 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate and 0.17 g/L anhydrous calcium chloride, with natural pH and water as a solvent.

Formulation of a fermentation medium: 50 g/L glucose, 12 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate, and 0.17 g/L anhydrous calcium chloride, with natural pH and water as a solvent.

Formulation of a solid medium: 50 g/L glucose, 12 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate, 0.17 g/L anhydrous calcium chloride and 10 g/L agar powder, with natural pH and water as a solvent.

### Test Method:

Proportion detection methods for DHA , DPA and EPA were carried out with reference to GB 5009.168-2016, and a fatty acid area normalization method was used for calculation.

### Example 1: Screening of Schizochytrium sp.

### 1. Mutagenesis of Schizochytrium sp.

The seed strains of Schizochytrium sp. HS01 (deposited at the China General Microbiological Culture Collection Center (CGMCC) via the deposit number of CGMCC No. 13746) were inoculated into the seed medium at an inoculum size of 2%, and cultured on a shaker at 28°C and 180 r/min for 24 hours to obtain a seed suspension. 10 mL of the seed suspension was pipetted, and centrifuged at 5000 r/min for 3 minutes, and the supernatant was discarded. The pellet was washed twice repeatedly with a phosphate buffer at pH 8.0 and resuspended. The seed suspension was added to a liquid seed medium containing 1.0% ethyl methanesulfonate and cultured at 28°C and 180 r/min for 6 hours.

### 2. Screening of Schizochytrium sp.

4 mL of the above mutated Schizochytrium solution was pipetted and diluted with a phosphate buffer at pH 8.0 to gradients of 10⁻², 10⁻³, 10⁻⁴ and 10⁻⁵. 200 µL of the 10⁻⁵ gradient diluted mutated Schizochytrium solution was pipetted and spread onto solid media containing 0.02 mM and 0.08 mM quizalofop-p-ethyl, respectively, and cultured upside down in an incubator at 37°C for 48 hours. Single colonies with appropriate sizes and regular edges were picked and streaked again onto solid media containing 0.02 mM and 0.08 mM quizalofop-p-ethyl, followed by incubation at 37°C for 48 hours.

### 3. Fermentation culture of Schizochytrium sp.

3 strains with relatively fast growth rates were selected and inoculated into seed media, followed by cultivation at 28°C and 180 r/min for 48 hours to obtain the inoculum, which were then preserved. The obtained inoculums was inoculated into fermentation media at an inoculum size of 4% and cultured for 120 hours at a cultivation temperature of 28°C and a stirring speed of 180 r/min to obtain a fermentation broth. The fermentation broth was then collected to extract oil, and the proportions of EPA, n-3 DPA and DHA in the total fatty acids of the obtained fermentation broth were determined. The results are shown in Table 1.

**Table 1: Proportions of EPA, n-3 DPA and DHA in Total Fatty Acids of Obtained Fermentation Broth**

| Concentration of quizalofop-p-ethyl (mM) | Strain No. | EPA (%) | n-3DPA (%) | DHA (%) |
|---|---|---|---|---|
| Control group (HS01) | - | 0.77 | 0.42 | 62.83 |
| 0.02 | 1-1 | 5.5 | 4.38 | 43.05 |
| 0.02 | 1-2 | 5.92 | 4.24 | 43.05 |
| 0.02 | 1-3 | 5.69 | 4.08 | 44.05 |
| 0.08 | 2-1 | 5.98 | 4.87 | 42.48 |
| 0.08 | 2-2 | 6.10 | 4.56 | 41.95 |
| 0.08 | 2-3 | 5.85 | 5.22 | 42.65 |

### Example 2

### 1. Mutagenesis of Schizochytrium sp.

10 mL of the seed suspension obtained in Example 1 was pipetted, and centrifuged at 5000 r/min for 3 minutes, and the supernatant was discarded. The pellet was washed twice with a phosphate buffer at pH 8.0 and resuspended. The seed suspension was then added to a fermentation medium containing 1.0% diethyl sulfate and cultured at 28°C and 180 r/min for 1 hour.

### 2. Screening of Schizochytrium sp.

4 mL of the above mutated Schizochytrium solution was pipetted and diluted with a phosphate buffer at pH 8.0 to gradients of 10⁻², 10⁻³, 10⁻⁴ and 10⁻⁵, respectively. 200 µL of the 10⁻⁵ gradient diluted mutated Schizochytrium solution was pipetted and spread onto a solid medium containing 0.01 mM cerulenin, and cultured upside down in an incubator at 37°C for 48 hours. Single colonies with appropriate sizes and regular edges were picked and streaked again onto a solid medium containing 0.01 mM cerulenin, followed by incubation at 37°C for 48 hours.

### 3. Fermentation culture of Schizochytrium sp.

3 strains with relatively fast growth rates were selected and fermented in the fermentation culture method described in step 3 of fermentation culture of Schizochytrium sp. in Example 1 to obtain a fermentation broth. The fermentation broth was then collected, and the oil in the fermentation broth was extracted with reference to the "hydrolysis-extraction method" in GB 5009.168-2016. The proportions of EPA, n-3 DPA and DHA in the total fatty acids of the obtained fermentation broth were determined. The results are shown in Table 2.

**Table 2: Proportions of EPA, n-3 DPA and DHA in Total Fatty Acids of Obtained Fermentation Broth**

| Concentration of cerulenin (mM) | Strain No. | EPA (%) | n-3DPA (%) | DHA (%) |
|---|---|---|---|---|
| Control group (HS01) | - | 0.77 | 0.42 | 62.83 |
| 0.01 | 3-1 | 6.38 | 5.52 | 41.07 |
| 0.01 | 3-2 | 5.80 | 4.10 | 44.7 |
| 0.01 | 3-3 | 5.50 | 4.38 | 43.05 |

### Example 3

The seed strain 3-1 in Example 2 was inoculated into the seed medium at an inoculum size of 2%, and cultured on a shaker at 28°C and 180 r/min for 24 hours to obtain a seed suspension.

### 1. Screening of Schizochytrium sp.

4 mL of the mutagenized Schizochytrium suspension was pipetted and diluted with a phosphate buffer at pH 8.0 to gradients of 10⁻², 10⁻³, 10⁻⁴ and 10⁻⁵. 200 µL of the 10⁻⁵ gradient diluted mutated Schizochytrium solution was pipetted and spread onto solid media containing 0.01 mM cerulenin and 0.08 mM quizalofop-p-ethyl, and cultured upside down in an incubator at 37°C for 48 hours. Single colonies with appropriate sizes and regular edges were picked and streaked again onto solid media containing 0.01 mM cerulenin and 0.08 mM quizalofop-p-ethyl, followed by incubation at 37°C for 48 hours.

### 2. Fermentation culture of Schizochytrium sp.

3 strains with relatively fast growth rates were selected and fermented in the fermentation culture method described in step 3 of fermentation culture of Schizochytrium sp. in Example 1. The fermentation broth was then collected, and the oil in the fermentation broth was extracted with reference to the "hydrolysis-extraction method" in GB 5009.168-2016. The proportions of EPA, n-3 DPA and DHA in the total fatty acids of the obtained fermentation broth were determined. The results are shown in Table 3.

**Table 3: Proportions of EPA, n-3 DPA and DHA in Total Fatty Acids of Obtained Fermentation Broth**

| Strain No. | EPA (%) | n-3DPA (%) | DHA (%) |
|---|---|---|---|
| 4-1 | 5.85 | 4.37 | 46.05 |
| 4-2 | 5.93 | 4.54 | 46.65 |
| 4-3 | 5.95 | 4.32 | 46.74 |

### Example 4

The seed strain 3-1 in Example 2 was inoculated into the seed medium at an inoculum size of 2%, and cultured on a shaker at 28°C and 180 r/min for 24 hours to obtain a seed suspension.

### 1. Screening of Schizochytrium sp.

4 mL of the mutagenized Schizochytrium suspension was pipetted and diluted with a phosphate buffer at pH 8.0 to gradients of 10⁻², 10⁻³, 10⁻⁴ and 10⁻⁵. 200 µL of the 10⁻⁵ gradient diluted mutated Schizochytrium solution was pipetted and spread onto solid media containing 0.01 mM cerulenin and 0.01 µg/L VB12 (Vitamin B12), and cultured in an incubator at 37°C for 48 hours. Single colonies with appropriate sizes and regular edges were picked and streaked again onto solid media containing 0.01 mM cerulenin and 0.01 µg/L VB12, followed by incubation at 37°C for 48 hours.

### 2. Fermentation culture of Schizochytrium sp.

3 strains with relatively fast growth rates were selected and fermented with reference to the fermentation culture method described in step 3 of fermentation culture of Schizochytrium sp. in Example 1 (in the strain preservation step after seed culture, strain 5-1 was named Schizochytrium sp. HS08, which has been deposited at the China General Microbiological Culture Collection Center (CGMCC) under the accession number CGMCC No. 40902) to obtain a fermentation broth. Oil in the fermentation broth was extracted with reference to the hydrolysis-extraction method in GB 5009.168-2016, and the proportions of EPA, n-3 DPA and DHA in the total fatty acids of the obtained fermentation broth were determined. The results are shown in Table 4.

**Table 4: Proportions of EPA, n-3 DPA and DHA in Total Fatty Acids of Obtained Fermentation Broth**

| Strain No. | EPA (%) | n-3DPA (%) | DHA (%) |
|---|---|---|---|
| 5-1 | 9.85 | 5.81 | 40.22 |
| 5-2 | 9.65 | 6.14 | 39.82 |
| 5-3 | 9.80 | 5.87 | 40.16 |

As shown in Table 1 and Table 2, the strains obtained by mutagenesis and screening in the present invention successfully increase the proportions of EPA and n-3 DPA in the total fatty acids of Schizochytrium sp. In Example 4, the strains obtained via mutagenesis and screening were further subjected to directed screening by using cerulenin combined with VB12. The proportion of EPA in the total fatty acids of the fermentation broth produced by strain 5-1 obtained from the directed screening was increased to 9.85%, and the proportion of n-3 DPA in the total fatty acids was increased to 5.81% (see Table 4), thereby exhibiting excellent and unexpected technical effects. Strain 5-1 was subjected to multiple subcultures, and all the obtained strains could stably produce various n-3 fatty acids. Strain 5-1 was named Schizochytrium sp. HS08, which has been deposited at the China General Microbiological Culture Collection Center (CGMCC) via the deposit number of CGMCC No. 40902. The strain exhibits excellent genetic stability, a fast growth rate, and promising prospects for industrial application.
The methods in the present invention are described by way of preferred embodiments. A person skilled in the art can obviously make modifications, or appropriate alterations and combinations to the methods and applications described herein within the content, spirit and scope of the present invention, to implement and apply the technology in the present invention. A person skilled in the art can appropriately improve the process parameters for implementation with reference to the content herein. It should be particularly noted that all similar substitutions and modifications are obvious to a person skilled in the art and are deemed to fall within the scope of the present invention.

## Claims

1. A strain of Schizochytrium sp., designated as Schizochytrium sp. HS08, with deposit information: deposited at the China General Microbiological Culture Collection Center (CGMCC) under the accession number CGMCC No. 40902.

2. A fermentation broth, prepared by fermenting the Schizochytrium sp. according to claim 1.

3. The fermentation broth according to claim 2, wherein a proportion of EPA in total fatty acids of the fermentation broth is not less than 9%; a proportion of n-3 DPA in the total fatty acids of the fermentation broth is not less than 5%; and/or a proportion of DHA in the total fatty acids of the fermentation broth is not less than 35%.

4. The fermentation broth according to claim 2, wherein a proportion of EPA in total fatty acids of the fermentation broth is 9% to 10%; a proportion of n-3 DPA in the total fatty acids of the fermentation broth is 5% to 7%; and/or a proportion of DHA in the total fatty acids of the fermentation broth is 35% to 45%.

5. A Schizochytrium suspension, wherein the Schizochytrium suspension comprises the Schizochytrium sp. according to claim 1.

6. A microbial agent, wherein the microbial agent comprises at least one of the Schizochytrium sp. according to claim 1, the fermentation broth according to any one of claims 2 to 4, and the Schizochytrium suspension according to claim 5.

7. A method for preparing the fermentation broth according to any one of claims 2 to 4, comprising: subjecting, to seed culture and fermentation culture, the Schizochytrium sp. according to claim 1.

8. The preparation method according to claim 7, wherein the seed culture comprises: culturing the Schizochytrium sp. HS08 in a seed medium to obtain an inoculum; and/or
the fermentation culture comprises: adding the inoculum obtained after the seed culture of the Schizochytrium sp. HS08 into a fermentation medium for fermentation culture;
optionally, the seed medium comprises the following components: 50 g/L glucose, 10 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate and 0.17 g/L anhydrous calcium chloride;
optionally, the fermentation medium comprises the following components: 50 g/L glucose, 12 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate, 0.17 g/L anhydrous calcium chloride and 10 g/L agar powder;
optionally, culture time for the seed culture is 12 to 60 hours or 48 hours; and
optionally, culture time for the fermentation culture is 100 to 300 hours or 120 hours.

9. A method for preparing an n-3 fatty acid, comprising: extracting the n-3 fatty acid from the fermentation broth according to claims 2 to 4 or the fermentation broth prepared in the preparation method according to claim 7 or 8, wherein
optionally, the n-3 fatty acid comprises at least one of EPA, n-3 DPA and DHA.

10. An n-3 fatty acid prepared in the preparation method according to claim 9.

11. A method for preparing microbial oil containing an n-3 fatty acid, comprising: extracting microbial oil containing an n-3 fatty acid from the fermentation broth according to claims 2 to 4 or the fermentation broth prepared in the preparation method according to claim 7 or 8, wherein
optionally, the microbial oil containing an n-3 fatty acid comprises at least one of EPA, n-3 DPA and DHA.

12. A microbial oil containing an n-3 fatty acid prepared in the preparation method according to claim 11.

13. Use of the Schizochytrium sp. according to claim 1, the fermentation broth according to any one of claims 2 to 4, the Schizochytrium suspension according to claim 5, the microbial agent according to claim 6, the fermentation broth prepared in the preparation method according to claim 7 or 8, the n-3 fatty acid prepared in the preparation method according to claim 9, the n-3 fatty acid according to claim 10, the microbial oil containing an n-3 fatty acid prepared in the preparation method according to claim 11, or the microbial oil containing an n-3 fatty acid according to claim 12 in the preparation of food, a cosmetic, a health product, feed and/or a medicament.

14. A mutagenic medium for mutagenizing a Schizochytrium sp., wherein the mutagenic medium contains a mutagen selected from one or more of nitrosoguanidine, ethyl methanesulfonate and diethyl sulfate; and a concentration of the mutagen is 0.1% to 5.0%.

15. The mutagenic medium according to claim 14, wherein the mutagen is 0.5% to 2% ethyl methanesulfonate or 0.5% to 2% diethyl sulfate; or preferably, the mutagen is 1% ethyl methanesulfonate or 1% diethyl sulfate.

16. The mutagenic medium according to claim 14, wherein the mutagenic medium further contains 40-60 g/L glucose, 9-15 g/L yeast extract powder, 15-20 g/L sodium sulfate anhydrous, 0.5-1 g/L potassium chloride, 3-5 g/L magnesium sulfate heptahydrate, 0.25-0.9 g/L potassium sulfate, 0.5-3.5 g/L potassium dihydrogen phosphate, 0.5-3.5 g/L ammonium sulfate and 0.1-2 g/L anhydrous calcium chloride, with natural pH; or preferably, the mutagenic medium contains 50 g/L glucose, 10 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate and 0.17 g/L anhydrous calcium chloride.

17. A screening medium for screening a Schizochytrium sp., wherein the screening medium contains a screening agent selected from one or more of quizalofop-p-ethyl, cerulenin, VB12 and diphenylamine; and a concentration of cerulenin is 0.001-1 mM, a concentration of quizalofop-p-ethyl is 0.001-1 mM, a concentration of diphenylamine is 0.001-1 mM, and a concentration of VB12 is 1-100 µg/L.

18. The screening medium according to claim 17, wherein the concentration of quizalofop-p-ethyl is 0.01-0.08 mM; the concentration of cerulenin is 0.01-0.02 mM; and the concentration of VB12 is 0.01 µg/L.

19. The screening medium according to claim 17 or 18, wherein the screening medium further contains 40-60 g/L glucose, 9-15 g/L yeast extract powder, 15-20 g/L sodium sulfate anhydrous, 0.5-1 g/L potassium chloride, 3-5 g/L magnesium sulfate heptahydrate, 0.25-0.9 g/L potassium sulfate, 0.5-3.5 g/L potassium dihydrogen phosphate, 0.5-3.5 g/L ammonium sulfate, 0.1-2 g/L anhydrous calcium chloride, and 5-20 g/L agar powder, with natural pH; or preferably, the screening medium contains 50 g/L glucose, 12 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate, 0.17 g/L anhydrous calcium chloride, and 10 g/L agar powder.

20. A method for screening a Schizochytrium sp., comprising steps of:
(1) inoculating the Schizochytrium sp. into a seed medium for cultivation for 24 hours to obtain a Schizochytrium inoculum;
(2) pipetting and centrifuging the seed solution and discarding a supernatant; washing and resuspending a residue with a phosphate buffer at pH 8.0 to obtain a seed suspension; and inoculating the seed suspension into the mutagenic medium according to any one of claims 14 to 16 to obtain a mutated Schizochytrium solution;
(3) pipetting the mutated Schizochytrium solution, and diluting the mutated Schizochytrium solution to a certain gradient with a phosphate buffer at pH 8.0, and then pipetting the diluted mutated Schizochytrium solution to spread on the screening medium according to any one of claims 17 to 19 for cultivation;
(4) selecting single colonies with fast growth rates and regular edges, and streaking the single colonies onto the screening medium according to any one of claims 17 to 19 for cultivation; and
(5) selecting colonies with fast growth rates and moderate size, inoculating the colonies into a seed medium, culturing for 24 hours and then preserving, to obtain the candidate purified high-yield Schizochytrium strain;
optionally, the seed medium contains 40-60 g/L glucose, 9-15 g/L yeast extract powder, 15-20 g/L sodium sulfate anhydrous, 0.5-1 g/L potassium chloride, 3-5 g/L magnesium sulfate heptahydrate, 0.25-0.9 g/L potassium sulfate, 0.5-3.5 g/L potassium dihydrogen phosphate, 0.5-3.5 g/L ammonium sulfate and 0.1-2 g/L anhydrous calcium chloride, with natural pH; or preferably, the seed medium contains 50 g/L glucose, 12 g/L yeast extract powder, 15 g/L sodium sulfate anhydrous, 0.5 g/L potassium chloride, 4.1 g/L magnesium sulfate heptahydrate, 0.65 g/L potassium sulfate, 1.0 g/L potassium dihydrogen phosphate, 1.0 g/L ammonium sulfate and 0.17 g/L anhydrous calcium chloride;
optionally, in step (2), the colonies are cultured at 28°C and 180 r/min for 1-6 hours;
in step (3), the colonies are cultured upside down in an incubator at 37°C for 48 hours;
in step (4), the colonies are cultured upside down in an incubator at 37°C for 48 hours; and/or in step (5), the colonies are cultured on a shaker at 28°C and 180 r/min for 24 hours.
